(19) Europäisches Patentamt

European Patent Office

Office européen des brevets

(11) **EP 1 674 118 A2**

(12) **EUROPEAN PATENT APPLICATION**

(43) Date of publication:
**28.06.2006 Bulletin 2006/26**

(51) Int Cl.:
*A61L 31/02* *(2006.01)*

(21) Application number: **05257453.0**

(22) Date of filing: **02.12.2005**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(30) Priority: **10.12.2004 US 9589**

(71) Applicant: **Cordis Corporation Miami Lakes, FL 33014 (US)**

(72) Inventors:
• **Burgermeister, Robert Bridgewater, NJ 08807 (US)**
• **Dave, Vipul Hillsborough, NJ 08844 (US)**
• **Grishaber, Randy-David Burce Asbury NJ 08802 (US)**

(74) Representative: **Belcher, Simon James Urquhart-Dykes & Lord LLP Tower North Central Merrion Way Leeds LS2 8PA (GB)**

(54) **Improved material for flexible connectors in high strength, high flexibility, controlled recoil stent**

(57)    A biocompatible material may be configured into any number of implantable medical devices including intraluminal stents. The biocompatible material may comprise metallic and non-metallic materials. These materials may be designed with a microstructure that facilitates or enables the design of devices with a wide range of geometries adaptable to various loading conditions.

In one embodiment an intraluminal scaffold is described. It comprises at least one flexible connector element having a luminal surface and an abluminal surface, the flexible connector element having a predetermined wall thickness, wherein the wall thickness is defined by the radial distance between the luminal surface and the abluminal surface, and a predetermined feature width, wherein an area bounded by the wall thickness and the feature width comprises a plurality of zones undergoing at least one of tensile, compressive or substantially zero stress change due to external loading, the flexible connector element being fabricated from a metallic material processed to have a microstructure with a granularity of about 32 microns or less and at least one internal grain boundary within the bounded area.

EP 1 674 118 A2

**Description**

The present invention relates to novel geometries for use in implantable medical devices, and more particularly, to novel stent designs manufactured or fabricated from alloys that provide high strength, high flexibility, high expansion capability, high fatigue resistance and controlled recoil. The present invention also relates to biocompatible materials, metallic and non-metallic, that provide for designed in microstructures that facilitate the design of devices with a wide range of geometries that are adaptable to various loading conditions.

Currently manufactured intravascular stents do not adequately provide sufficient tailoring of the microstructural properties of the material forming the stent to the desired mechanical behavior of the device under clinically relevant in-vivo loading conditions. Any intravascular device should preferably exhibit certain characteristics, including maintaining vessel patency through a chronic outward force that will help to remodel the vessel to its intended luminal diameter, preventing excessive radial recoil upon deployment, exhibiting sufficient fatigue resistance and exhibiting sufficient ductility so as to provide adequate coverage over the full range of intended expansion diameters.

Accordingly, there is a need to develop precursory materials and the associated processes for manufacturing intravascular stents that provide device designers with the opportunity to engineer the device to specific applications.

The present invention overcomes the limitations of applying conventionally available materials to specific intravascular therapeutic applications as briefly described above.

In accordance with one aspect, the present invention is directed to an intraluminal scaffold. The intraluminal scaffold comprises at least one flexible connector element having a luminal surface and an abluminal surface, the flexible connector element having a predetermined wall thickness, wherein the wall thickness is defined by the radial distance between the luminal surface and the abluminal surface, and a predetermined feature width, wherein an area bounded by the wall thickness and the feature width comprises a plurality of zones undergoing at least one of tensile, compressive or substantially zero stress change due to external loading, the flexible connector element being fabricated from a metallic material processed to have a microstructure with a granularity of about 32 microns or less and at least one internal grain boundary within the bounded area.

In accordance with another aspect, the present invention is directed to an intraluminal scaffold. The intraluminal scaffold comprises at least one flexible connector element having a luminal surface and an abluminal surface, the flexible connector element having a predetermined wall thickness, wherein the wall thickness is defined by the radial distance between the luminal surface and the abluminal surface, and a predetermined feature width, wherein an area bounded by the wall thickness and the feature width comprises a plurality of zones undergoing at least one of tensile, compressive or substantially zero stress change due to external loading, the flexible connector element being fabricated from a non-metallic material processed to have a microstructure with structural domains having a size of about 50 microns or less and at least one internal structural boundary within the bounded area.

The biocompatible material for implantable medical devices of the present invention offers a number of advantages over currently utilized materials. The biocompatible material of the present invention is magnetic resonance imaging compatible, is less brittle than other metallic materials, has enhanced ductility and toughness, and has increased durability. The biocompatible material also maintains the desired or beneficial characteristics of currently available metallic materials, including strength and flexibility.

The biocompatible material for implantable medical devices of the present invention may be utilized for any number of medical applications, including vessel patency devices such as vascular stents, biliary stents, ureter stents, vessel occlusion devices such as atrial septal and ventricular septal occluders, patent foramen ovale occluders and orthopedic devices such as fixation devices.

The biocompatible material of the present invention is simple and inexpensive to manufacture. The biocompatible material may be formed into any number of structures or devices. The biocompatible alloy may be thermomechanically processed, including cold-working and heat treating, to achieve varying degrees of strength and ductility. The biocompatible material of the present invention may be age hardened to precipitate one or more secondary phases.

The intraluminal stent of the present invention may be specifically configured to optimize the number of discrete equiaxed grains that comprise the wall dimension so as to provide the intended user with a high strength, controlled recoil device as a function of expanded inside diameter.

The biocompatible material of the present invention comprises a unique composition and designed-in properties that enable the fabrication of stents that are able to withstand a broader range of loading conditions than currently available stents. More particularly, the microstructure designed into the biocompatible material facilitates the design of stents with a wide range of geometries that are adaptable to various loading conditions.

The biocompatible materials of the present invention also include non-metallic materials, including polymeric materials. These non-metallic materials may be designed to exhibit properties substantially similar to the metallic materials described herein, particularly with respect to the microstructure design, including the presence of at least one internal grain boundary or its non-metallic equivalent; namely, spherulitic boundary.

Embodiments of the invention will now be described by way of example with reference to the accompanying drawings, in which:

Figure 1 is a graphical representation of the transition of critical mechanical properties as a function of thermomechanical processing for cobalt-chromium alloys in accordance with the present invention;

Figure 2 is a graphical representation of the endurance limit chart as a function of thermomechanical processing for a cobalt-chromium alloy in accordance with the present invention;

Figure 3 is a planar representation of an exemplary stent fabricated from the biocompatible alloy in accordance with the present invention;

Figure 4 is a detailed planar representation of a hoop of an exemplary stent fabricated from the biocompatible alloy in accordance with the present invention;

Figure 5 is a simplified schematic cross-sectional representation of a load bearing intraluminal scaffold element in accordance with the present invention;

Figure 6 is a first simplified schematic cross-sectional representation of a flexible connector intraluminal scaffold element in accordance with the present invention;

Figure 7 is a second simplified schematic cross-sectional representation of a flexible connector intraluminal scaffold element in accordance with the present invention; and

Figure 8 is a third simplified schematic cross-sectional representation of a flexible connector intraluminal scaffold element in accordance with the present invention.

Biocompatible, solid-solution strengthened alloys such as iron-based alloys, cobalt-based alloys and titanium-based alloys as well as refractory metals and refractory-based alloys may be utilized in the manufacture of any number of implantable medical devices. The biocompatible alloy for implantable medical devices in accordance with the present invention offers a number of advantages over currently utilized medical grade alloys. The advantages include the ability to engineer the underlying microstructure in order to sufficiently perform as intended by the designer without the limitations of currently utilized materials and manufacturing methodologies.

For reference, a traditional stainless steel alloy such as 316L (i.e. UNS S31603) which is broadly utilized as an implantable, biocompatible device material may comprise chromium (Cr) in the range from about 16 to 18 wt.%, nickel (Ni) in the range from about 10 to 14 wt.%, molybdenum (Mo) in the range from about 2 to 3 wt.%, manganese (Mn) in the range up to 2 wt.%, silicon (Si) in the range up to 1 wt.%, with iron (Fe) comprising the balance (approximately 65 wt.%) of the composition.

Additionally, a traditional cobalt-based alloy such as L605 (i.e. UNS R30605) which is also broadly utilized as an implantable, biocompatible device material may comprise chromium (Cr) in the range from about 19 to 21 wt.%, tungsten (W) in the range from about 14 to 16 wt.%, nickel (Ni) in the range from about 9 to 11 wt.%, iron (Fe) in the range up to 3 wt.%, manganese (Mn) in the range up to 2 wt.%, silicon (Si) in the range up to 1 wt.%, with cobalt (cobalt) comprising the balance (approximately 49 wt.%) of the composition.

Alternately, another traditional cobalt-based alloy such as Haynes 188 (i.e. UNS R30188) which is also broadly utilized as an implantable, biocompatible device material may comprise nickel (Ni) in the range from about 20 to 24 wt.%, chromium (Cr) in the range from about 21 to 23 wt.%, tungsten (W) in the range from about 13 to 15 wt.%, iron (Fe) in the range up to 3 wt.%, manganese (Mn) in the range up to 1.25 wt.%, silicon (Si) in the range from about 0.2 to 0.5 wt.%, lanthanum (La) in the range from about 0.02 to 0.12 wt.%, boron (B) in the range up to 0.015 wt.% with cobalt (Co) comprising the balance (approximately 38 wt.%) of the composition.

In general, elemental additions such as chromium (Cr), nickel (Ni), tungsten (W), manganese (Mn), silicon (Si) and molybdenum (Mo) were added to iron- and/or cobalt-based alloys, where appropriate, to increase or enable desirable performance attributes, including strength, machinability and corrosion resistance within clinically relevant usage conditions.

In accordance with one exemplary embodiment, a cobalt-based alloy may comprise from about nil to about metallurgically insignificant trace levels of elemental iron (Fe) and elemental silicon (Si), elemental iron only, or elemental silicon only. For example, the cobalt-based alloy may comprise chromium in the range from about 10 weight percent to about 30 weight percent, tungsten in the range from about 5 weight percent to about 20 weight percent, nickel in the range from about 5 weight percent to about 20 weight percent, manganese in the range from about 0 weight percent to about 5 weight percent, carbon in the range from about 0 weight percent to about 1 weight percent, Iron in an amount not to exceed 0.12 weight percent, silicon in an amount not to exceed 0.12 weight percent, phosphorus in an amount not to exceed 0.04 weight percent, sulfur in an amount not to exceed 0.03 weight percent and the remainder cobalt. Alternately, the cobalt-based alloy may comprise chromium in the range from about 10 weight percent to about 30 weight percent, tungsten in the range from about 5 weight percent to about 20 weight percent, nickel in the range from about 5 weight percent to about 20 weight percent, manganese in the range from about 0 weight percent to about 5 weight percent, carbon in the range from about 0 weight percent to about 1 weight percent, iron in an amount not to exceed 0.12 weight percent, silicon in an amount not to exceed 0.4 weight percent, phosphorus in an amount not to exceed 0.04 weight percent, sulfur in an amount not to exceed 0.03 weight percent and the remainder cobalt. In yet another alternative composition, the cobalt-based alloy may comprise chromium in the range from about 10 weight percent to about 30 weight percent, tungsten in the range from about 5 weight percent to about 20 weight percent, nickel in the range from about 5 weight percent to about 20 weight percent, manganese in the range from about 0 weight percent to about 5 weight percent, carbon in the range from about 0 weight percent to about 1 weight percent, iron in an amount not to exceed 3 weight percent, silicon in an amount not to exceed 0.12 weight percent, phosphorus in an amount not to exceed 0.04 weight percent, sulfur in an amount not to exceed 0.03 weight percent and the remainder cobalt.

In accordance with an exemplary embodiment, an implantable medical device may be formed from a solid-solution alloy comprising nickel in the range from about 20 weight percent to about 24 weight percent, chromium in the range from about 21 weight percent to about 23 weight percent, tungsten in the range from about 13 weight percent to about 15 weight percent, manganese in the range from about 0 weight percent to about 1.25 weight percent, carbon in the range from about 0.05 weight percent to about 0.15 weight percent, lanthanum in the range from about 0.02 weight percent to about 0.12 weight percent, boron in the range from about 0 weight percent to about 0.015 weight percent, iron in an amount not to exceed 0.12 weight percent, silicon in an amount not to exceed 0.12 weight percent and the remainder cobalt.

In accordance with another exemplary embodiment, an implantable medical device may be formed from a solid-solution alloy comprising nickel in the range from about 20 weight percent to about 24 weight percent, chromium in the range from about 21 weight percent to about 23 weight percent, tungsten in the range from about 13 weight percent to about 15 weight percent, manganese in the range from about 0 weight percent to about 1.25 weight percent, carbon in the range from about 0.05 weight percent to about 0.15 weight percent, lanthanum in the range from about 0.02 weight percent to about 0.12 weight percent, boron in the range from about 0 weight percent to about 0.015 weight percent, silicon in the range from about 0.2 weight percent to about 0.5 weight percent, iron in an amount not to exceed 0.12 weight percent and the remainder cobalt

In accordance with yet another exemplary embodiment, an implantable medical device may be formed from a solid-solution alloy comprising nickel in the range from about 20 weight percent to about 24 weight percent, chromium in the range from about 21 weight percent to about 23 weight percent, tungsten in the range from about 13 weight percent to about 15 weight percent, iron in the range from about 0 weight percent to about 3 weight percent, manganese in the range from about 0 weight percent to about 1.25 weight percent, carbon in the range from about 0.05 weight percent to about 0.15 weight percent, lanthanum in the range from about 0.02 weight percent to about 0.12 weight percent, boron in the range from about 0 weight percent to about 0.015 weight percent, silicon in an amount not to exceed 0.12 weight percent and the remainder cobalt.

In contrast to the traditional formulation of this alloy (i.e. Alloy 188 / Haynes 188), the intended composition does not include any elemental iron (Fe) or silicon (Si) above conventional accepted trace impurity levels. Accordingly, this exemplary embodiment will exhibit a marked reduction in 'susceptibility' (i.e. the magnetic permeability) thereby leading to improved magnetic resonance imaging compatibility. Additionally, the exemplary embodiment will exhibit a marked improvement in material ductility and fatigue strength (i.e. cyclic endurance limit strength) due to the elimination of silicon (Si), above trace impurity levels.

The composition of the material of the present invention does not eliminate ferromagnetic components but rather shift the 'susceptibility' (i.e. the magnetic permeability) such that the magnetic resonance imaging compatibility may be improved. In addition, the material of the present invention is intended to improve the measurable ductility by minimizing the deleterious effects induced by traditional machining aides such as silicon (Si).

It is important to note that any number of alloys and engineered metals, including iron-based alloys, cobalt-based alloys, refractory-based alloys, refractory metals, and titanium-based alloys may be used in accordance with the present invention. However, for ease of explanation, a detailed description of a cobalt-based alloy will be utilized in the following detailed description.

An exemplary embodiment may be processed from the requisite elementary raw materials, as set-forth above, by first mechanical homogenization (i.e. mixing) and then compaction into a green state (i.e. precursory) form. If necessary, appropriate manufacturing aids such as hydrocarbon based lubricants and/or solvents (e.g. mineral oil, machine oils, kerosene, isopropanol and related alcohols) be used to ensure complete mechanical homogenization. Additionally, other processing steps such as ultrasonic agitation of the mixture followed by cold compaction to remove any unnecessary manufacturing aides and to reduce void space within the green state may be utilized. It is preferable to ensure that any impurities within or upon the processing equipment from prior processing and/or system construction (e.g. mixing vessel material, transfer containers, etc.) be sufficiently reduced in order to ensure that the green state form is not unnecessarily contaminated. This may be accomplished by adequate cleaning of the mixing vessel before adding the constituent elements by use of surfactant-based cleaners to remove any loosely adherent contaminants.

Initial melting of the green state form into an ingot of desired composition, is achieved by vacuum induction melting (VIM) where the initial form is inductively heated to above the melting point of the primary constituent elements within a refractory crucible and then poured into a secondary mold within a vacuum environment (e.g. typically less than or equal to 0.0133 Pa ($10^{-4}$mmHg)). The vacuum process ensures that atmospheric contamination is significantly minimized. Upon solidification of the molten pool, the ingot bar is substantially single phase (i.e. compositionally homogenous) with a definable threshold of secondary phase impurities that are typically ceramic (e.g. carbide, oxide or nitride) in nature. These impurities are typically inherited from the precursor elemental raw materials.

A secondary melting process termed vacuum arc reduction (VAR) is utilized to further reduce the concentration of the secondary phase impurities to a conventionally accepted trace impurity level (i.e. < 1,500 ppm). Other methods maybe enabled by those skilled in the art of ingot formulation that substantially embodies this practice of ensuring that atmospheric contamination is minimized. In addition, the initial VAR step may be followed by repetitive VAR processing to further homogenize the solid-solution alloy in the ingot form. From the initial ingot configuration, the homogenized alloy will be further reduced in product size and form by various industrially accepted methods such as, but not limited too, ingot peeling, grinding, cutting, forging, forming, hot rolling and/or cold finishing processing steps so as to produce bar stock that may be further reduced into a desired raw material form.

In this exemplary embodiment, the initial raw material product form that is required to initiate the thermomechanical processing that will ultimately yield a desired small diameter, thin-walled tube, appropriate for interventional devices, is a modestly sized round bar (e.g. 25.4 mm (one inch) in diameter round bar stock) of predetermined length. In order to facilitate the reduction of the initial bar stock into a much smaller tubing configuration, an initial clearance hole must be placed into the bar stock that runs the length of the product. These tube hollows (i.e. heavy walled tubes) may be created by 'gun-drilling' (i.e. high depth to diameter ratio drilling) the bar stock. Other industrially relevant methods of creating the tube hollows from round bar stock may be utilized by those skilled-in-the-art of tube making.

Consecutive mechanical cold-finishing operations such as drawing through a compressive outer-diameter (OD), precision shaped (i.e. cut), circumferentially complete, diamond die using any of the following internally supported (i.e. inner diameter, ID) methods, but not necessarily limited to these conventional forming methods, such as hard mandrel (i.e. relatively long traveling ID mandrel also referred to as rod draw), floating-plug (i.e. relatively short ID mandrel that 'floats' within the region of the OD compressive die and fixed-plug (i.e. the ID mandrel is 'fixed' to the drawing apparatus where relatively short workpieces are processed) drawing. These process steps are intended to reduce the outer-diameter (OD) and the corresponding wall thickness of the initial tube hollow to the desired dimensions of the finished product.

When necessary, tube sinking (i.e. OD reduction of the workpiece without inducing substantial tube wall reduction) is accomplished by drawing the workpiece through a compressive die without internal support (i.e. no ID mandrel). Conventionally, tube sinking is typically utilized as a final or near-final mechanical processing step to achieve the desired dimensional attributes of the finished product.

Although not practically significant, if the particular compositional formulation will support a single reduction from the initial raw material configuration to the desired dimensions of the finished product, in process heat-treatments will not be necessary. Where necessary to achieve intended mechanical properties of the finished product, a final heat-treating step is utilized.

Conventionally, all metallic alloys in accordance with the present invention will require incremental dimensional reductions from the initial raw material configuration to reach the desired dimensions of the finished product. This processing constraint is due to the material's ability to support a finite degree of induced mechanical damage per processing step without structural failure (e.g. strain-induced fracture, fissures, extensive void formation, etc.).

In order to compensate for induced mechanical damage (i.e. cold-working) during any of the aforementioned cold-finishing steps, periodic thermal heat-treatments are utilized to stress-relieve, (i.e. minimization of deleterious internal residual stresses that are the result of processes such as cold-working) thereby increasing the workability (i.e. ability to support additional mechanical damage without measurable failure) of the workpiece prior to subsequent reductions. These thermal treatments are typically, but not necessarily limited to, conducted within a relatively inert environment such as an inert gas furnace (e.g. nitrogen, argon, etc.), an oxygen rarified hydrogen furnace, a conventional vacuum furnace and under less common process conditions, atmospheric air. When vacuum furnaces are utilized, the level of vacuum (i.e. subatmospheric pressure), typically measured in units of mmHg or torr (where 1 mmHg is equal to 1 unit torr), shall be sufficient to ensure that excessive and deteriorative high temperature oxidative processes are not functionally operative during heat treatment. This process may usually be achieved under vacuum conditions of $10^{-4}$ mmHg (0.0001 torr) or less (i.e. lower magnitude).

The stress relieving heat treatment temperature is typically held constant between 82 to 86 percent of the conventional melting point (i.e. industrially accepted liquidus temperature, 0.82 to 0.86 homologous temperatures) within an adequately sized isothermal region of the heat-treating apparatus. The workpiece undergoing thermal treatment is held within the isothermal processing region for a finite period of time that is adequate to ensure that the workpiece has reached a state of thermal equilibrium and such that sufficient time has elapsed to ensure that the reaction kinetics (i.e. time dependent material processes) of stress-relieving and/or process annealing, as appropriate, has been adequately completed. The finite amount of time that the workpiece is held within the processing is dependent upon the method of bringing the workpiece into the process chamber and then removing the working upon completion of heat treatment. Typically, this process is accomplished by, but not limited to, use of a conventional conveyor-belt apparatus or other relevant mechanical assist devices. In the case of the former, the conveyor belt speed and appropriate finite dwell-time, as necessary, within the isothermal region is controlled to ensure that sufficient time at temperature is utilized so as to ensure that the process is completed as intended.

When necessary to achieve desired mechanical attributes of the finished product, heat-treatment temperatures and corresponding finite processing times may be intentionally utilized that are not within the typical range of 0.82 to 0.86 homologous temperatures. Various age hardening (i.e. a process that induces a change in properties at moderately elevated temperatures, relative to the conventional melting point, that does not induce a change in overall chemical composition within the metallic alloy being processed) processing steps may be carried out, as necessary, in a manner consistent with those previously described at temperatures substantially below 0.82 to 0.86 homologous temperature. For cobalt-based alloys in accordance with the present invention, these processing temperatures may be varied between and inclusive of approximately 0.29 homologous temperature and the aforementioned stress relieving temperature range. The workpiece undergoing thermal treatment is held within the isothermal processing region for a finite period of time that is adequate to ensure that the workpiece has reached a state of thermal equilibrium and for that sufficient time is elapsed to ensure that the reaction kinetics (i.e. time dependent material processes) of age hardening, as appropriate, is adequately completed prior to removal from the processing equipment.

In some cases for cobalt-based alloys in accordance with the present invention, the formation of secondary-phase ceramic compounds such as carbide, nitride and/or oxides will be induced or promoted by age hardening heat-treating. These secondary-phase compounds are typically, but not limited to, for cobalt-based alloys in accordance with the present invention, carbides which precipitate along thermodynamically favorable regions of the structural crystallographic planes that comprise each grain (i.e. crystallographic entity) that make-up the entire polycrystalline alloy. These secondary-phase carbides can exist along the intergranular boundaries as well as within each granular structure (i.e. intragranular). Under most circumstances for cobalt-based alloys in accordance with the present invention, the principal secondary phase carbides that are stoichiometrically expected to be present are $M_6C$ where M typically is cobalt (cobalt). When present, the intermetallic $M_6C$ phase is typically expected to reside intragranularly along thermodynamically favorable regions of the structural crystallographic planes that comprise each grain within the polycrystalline alloy in accordance with the present invention. Although not practically common, the equivalent material phenomena can exist for a single crystal (i.e. monogranular) alloy.

Additionally, another prominent secondary phase carbide can also be induced or promoted as a result of age hardening heat treatments. This phase, when present, is stoichiometrically expected to be $M_{23}C_6$ where M typically is chromium (Cr) but is also commonly observed to be cobalt (cobalt) especially in cobalt-based alloys. When present, the intermetallic $M_{23}C_6$ phase is typically expected to reside along the intergranular boundaries (i.e. grain boundaries) within a polycrystalline alloy in accordance with the present invention. As previously discussed for the intermetallic $M_6C$ phase, the equivalent presence of the intermetallic $M_{23}C_6$ phase can exist for a single crystal (i.e. monogranular) alloy, albeit not practically common.

In the case of the intergranular $M_{23}C_6$ phase, this secondary phase is conventionally considered most important, when formed in a manner that is structurally and compositionally compatible with the alloy matrix, to strengthening the grain boundaries to such a degree that intrinsic strength of the grain boundaries and the matrix are adequately balanced. By inducing this equilibrium level of material strength at the microstructural level, the overall mechanical properties of the finished tubular product can be further optimized to desirable levels.

In addition to stress relieving and age hardening related heat-treating steps, solutionizing (i.e. sufficiently high temperature and longer processing time to thermodynamically force one of more alloy constituents to enter into solid solution - 'singular phase', also referred to as full annealing) of the workpiece may be utilized. For cobalt-based alloys in accordance with the present invention, the typical solutionizing temperature can be varied between and inclusive of approximately 0.88 to 0.90 homologous temperatures. The workpiece undergoing thermal treatment is held within the isothermal processing region for a finite period of time that is adequate to ensure that the workpiece has reached a state of thermal equilibrium and for that sufficient time is elapsed to ensure that the reaction kinetics (i.e. time dependent material processes) of solutionizing, as appropriate, is adequately completed prior to removal from the processing equipment.

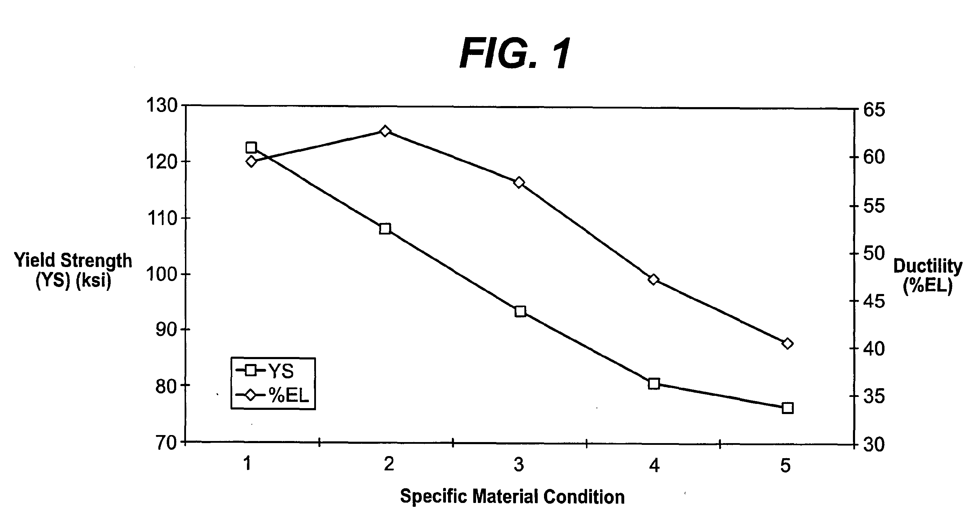

The sequential and selectively ordered combination of thermomechanical processing steps that may comprise but not necessarily include mechanical cold-finishing operations, stress relieving, age hardening and solutionizing can induce and enable a broad range of measurable mechanical properties as a result of distinct and determinable microstructural attributes. This material phenomena can be observed in Figure 1, which shows a chart that exhibits the affect of thermomechanical processing (TMP) such as cold working and in-process heat-treatments on measurable mechanical properties such as yield strength and ductility (presented in units of percent elongation) in accordance with the present invention. In this example, thermomechanical (TMP) groups one (1) through five (5) were subjected to varying combinations of cold-finishing, stress relieving and age hardening and not necessarily in the presented sequential order. In general, the principal isothermal age hardening heat treatment applied to each TMP group varied between about 0.74 to 0.78 homologous temperatures for group (1), about 0.76 to 0.80 homologous temperatures for group (2), about 0.78 to 0.82 homologous temperatures for group (3), about 0.80 to 0.84 homologous temperatures for group (4) and about 0.82 to 0.84 homologous temperatures for group (5). Each workpiece undergoing thermal treatment was held within the isothermal processing region for a finite period of time that was adequate to ensure that the workpiece reached a state of thermal equilibrium and to ensure that sufficient time was elapsed to ensure that the reaction kinetics of age hardening was adequately completed.

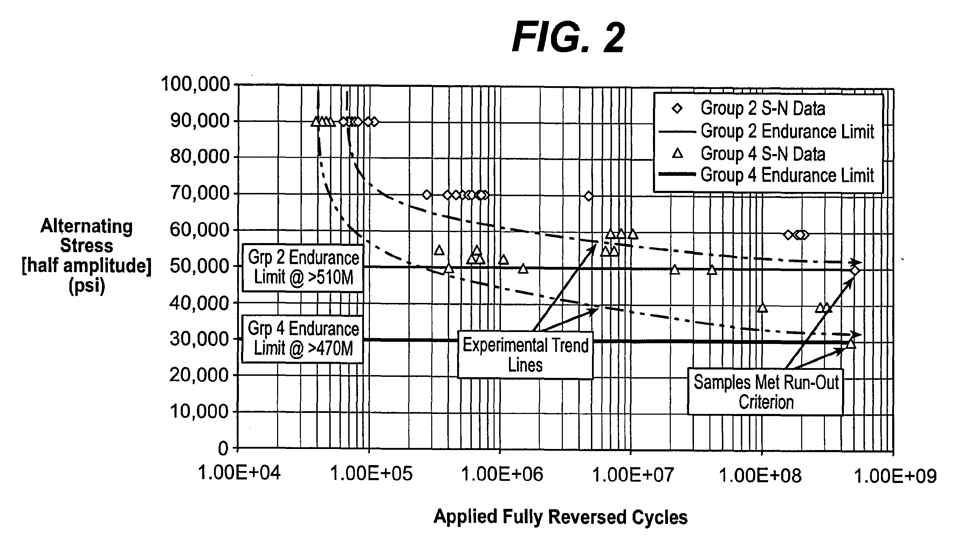

More so, the effect of thermomechanical processing (TMP) on cyclic fatigue properties is on cobalt-based alloys, in accordance with the present invention, is reflected in Figure 2. Examination of Figure 2, shows the affect on fatigue strength (i.e. endurance limit) as a function of thermomechanical processing for the previously discussed TMP groups (2) and (4). TMP group (2) from this figure as utilized in this specific example shows a marked increase in the fatigue strength (i.e. endurance limit, the maximum stress below which a material can presumably endure an infinite number of stress cycles) over and against the TMP group (4) process.

Other alloys may also be utilized in accordance with the present invention. For reference, a traditional cobalt-based alloy such as MP35N (i.e. UNS R30035) which is also broadly utilized as an implantable, biocompatible device material may comprise a solid-solution alloy comprising nickel in the range from about 33 weight percent to about 37 weight percent, chromium in the range from about 19 weight percent to about 21 weight percent, molybdenum in the range from about 9 weight percent to about 11 weight percent, iron in the range from about 0 weight percent to about 1 weight percent, titanium in the range from about 0 percent to about 1 weight percent, manganese in the range from about 0 weight percent to about 0.15 weight percent, silicon in the range from about 0 weight percent to about 0.15 percent, carbon in the range from about 0 to about 0.025 weigh percent, phosphorous in the range from about 0 to about 0.015 weight percent, boron in the range from about 0 to about 0.015 weight percent, sulfur in the range from about 0 to about 0.010 weight percent, and the remainder cobalt.

As described above, elemental additions such as chromium (Cr), nickel (Ni), manganese (Mn), silicon (Si) and molybdenum (Mo) were added to iron-and/or cobalt-based alloys, where appropriate, to increase or enable desirable performance attributes, including strength, machinability and corrosion resistance within clinically relevant usage conditions.

In accordance with an exemplary embodiment, an implantable medical device may be formed from a solid-solution alloy comprising nickel in the range from about 33 weight percent to about 37 weight percent, chromium in the range from about 19 weight percent to about 21 weight percent, molybdenum in the range from about 9 weight percent to about 11 weight percent, iron in the range from about 0 weight percent to about 1 weight percent, manganese in the range from about 0 weight percent to about 0.15 weight percent, silicon in the range from about 0 weight percent to about 0.15 weight percent, carbon in the range from about 0 weight percent to about 0.015 weight percent, phosphorous in the range from about 0 to about 0.015 weight percent, boron in the range from about 0 to about 0.015 weight percent, sulfur in the range from about 0 to about 0.010 weight percent, titanium in an amount not to exceed 0.015 weight percent and the remainder cobalt.

In contrast to the traditional formulation of MP35N, the intended composition does not include any elemental titanium (Ti) above conventional accepted trace impurity levels. Accordingly, this exemplary embodiment will exhibit a marked improvement in fatigue durability (i.e. cyclic endurance limit strength) due to the minimization of secondary phase precipitates in the form of titanium-carbides.

In accordance with another exemplary embodiment, an implantable medical device may be formed from a biocompatible, solid-solution alloy comprising chromium in the range from about 26 weight percent to about 30 weight percent, molybdenum in the range from about 5 weight percent to about 7 weight percent, nickel in the range from about 0 weight percent to about 1 weight percent, silicon in the range from about 0 weight percent to about 1 weight percent, manganese in the range from about 0 weight percent to about 1 weight percent, iron in the range from about 0 weight percent to about 0.75 weight percent, nitrogen in the range from about 0 to about 0.25 weight percent, carbon in an amount not to exceed 0.025 weight percent and the remainder cobalt.

These alloys may be processed similarly to the other alloys described herein, and exhibit similar characteristics. Once the all intended processing is complete, the tubular product may be configured into any number of implantable medical devices including intravascular stents, filters, occlusionary devices, shunts and embolic coils. In accordance with an exemplary embodiment of the present invention, the tubular product is configured into a stent or intraluminal scaffold. Preferred material characteristics of a stent include strength, fatigue robustness and sufficient ductility.

Strength is an intrinsic mechanical attribute of the raw material. As a result of prior thermomechanical processing, the resultant strength attribute can be assigned primarily to the underlying microstructure that comprises the raw material. The causal relationship between material structure, in this instance, grain-size, and the measurable strength, in this instance yield strength, is explained by the classical Hall-Petch relationship where strength is inversely proportional the square of grain-size as given by,

$$\sigma_y \propto \frac{1}{\sqrt{G.S.}} \qquad (1)$$

wherein $\sigma_y$ is the yield strength as measured in MPa and G.S. is grain-size as measured in millimeters as the average granular diameter. The strength attribute specifically affects the ability of the intravascular device to maintain vessel patency under *in-vivo* loading conditions.

The causal relationship between balloon-expandable device recoil (i.e. elastic "spring-back" upon initial unloading by deflation of the deployment catheter's balloon) and strength, in this instance yield strength, is principally affected by grain-size. As previously described, a decrement in grain-size results in higher yield strength as shown above. Accordingly, the measurable device recoil is inversely proportional to the grain-size of the material.

The causal relationship between fatigue resistance, in this instance endurance limit or the maximum stress below which a material can presumably endure an infinite number of stress cycles, and strength, in this instance yield strength, is principally affected by grain-size. Although fatigue resistance is also affected by extrinsic factors such as existing material defects, for example, stable cracks and processing flaws, the principal intrinsic factor affecting fatigue resistance for a given applied load is material strength. As previously described, a decrement in grain-size results in higher yield strength as shown above. Accordingly, the endurance limit (i.e. fatigue resistance) is inversely proportional to the grain-size of the material.

The causal relationship between ductility, in this instance the material's ability to support tensile elongation without observable material fracture (i.e. percent elongation), is significantly affected by grain-size. Typically, ductility is inversely proportional to strength that would imply a direct relationship to grain-size.

In accordance with the exemplary embodiment described herein, microstructural attributes, in this instance, grain-size, may be configured to be equal to or less than about 32 microns in average diameter. In order to ensure that all of the measurable mechanical attributes are homogenous and isotropic within the intended structure or stent, an equiaxed distribution of granularity is preferable. So as to ensure that the structural properties of the intended stent are configured in the preferred manner, a minimum of about two structurally finite intergranular elements (i.e. grains) to a maximum of about ten structurally finite intergranular elements shall exist within a given region of the stent components or elements. In particular, the number of grains may be measured as the distance between the abluminal and the luminal surface of the stent component (i.e. wall thickness). While these microstructural aspects may be tailored throughout the entirety of the stent, it may be particularly advantageous to configure the deformable regions of the stent with these microstructural aspects as described in detail below.

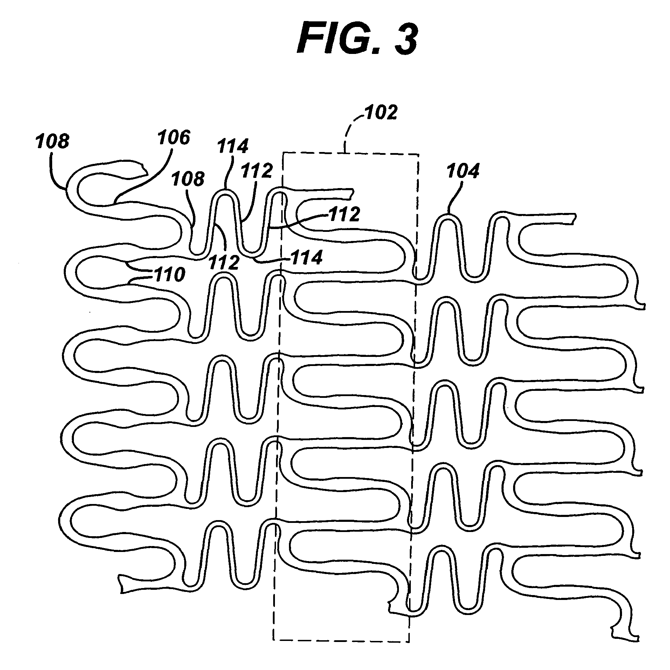

Referring to Figure 3, there is illustrated a partial planar view of an exemplary stent 100 in accordance with the present invention. The exemplary stent 100 comprises a plurality of hoop components 102 interconnected by a plurality of flexible connectors 104. The hoop components 102 are formed as a continuous series of substantially circumferentially oriented radial strut members 106 and alternating radial arc members 108. Although shown in planar view, the hoop components 102 are essentially ring members that are linked together by the flexible connectors 104 to form a substantially tubular stent structure. The combination of radial strut members 106 and alternating radial arc members 108 form a substantially sinusoidal pattern. Although the hoop components 102 may be designed with any number of design features and assume any number of configurations, in the exemplary embodiment, the radial strut members 106 are wider in their central regions 110. This design feature may be utilized for a number of purposes, including, increased surface area for drug delivery.

The flexible connectors 104 are formed from a continuous series of substantially longitudinally oriented flexible strut members 112 and alternating flexible arc members 114. The flexible connectors 104, as described above, connect adjacent hoop components 102 together. In this exemplary embodiment, the flexible connectors 104 have a substantially N-shape with one end being connected to a radial arc member on one hoop component and the other end being connected to a radial arc member on an adjacent hoop component. As with the hoop components 102, the flexible connectors 104 may comprise any number of design features and any number of configurations. In the exemplary embodiment, the ends of the flexible connectors 104 are connected to different portions of the radial arc members of adjacent hoop components for ease of nesting during crimping of the stent. It is interesting to note that with this exemplary configuration, the radial arcs on adjacent hoop components are slightly out of phase, while the radial arcs on every other hoop component are substantially in phase. In addition, it is important to note that not every radial arc on each hoop component need be connected to every radial arc on the adjacent hoop component.

The substantially tubular structure of the stent 100 provides the scaffolding for maintaining the patentcy of substantially tubular organs, such as arteries. The stent 100 comprises a luminal surface and an abluminal surface. The distance between the two surfaces defines the wall thickness as is described in detail above. The stent 100 has an unexpanded diameter for delivery and an expanded diameter which roughly corresponds to the normal diameter of the organ into which it is delivered. As tubular organs such as arteries may vary in diameter, different size stents having different sets of unexpanded and expanded diameters may be designed without departing from the scope of the present invention. As described herein, the stent 100 may be formed form any number of metallic materials, including cobalt-based alloys, iron-based alloys, titanium-based alloys, refractory-based alloys and refractory metals.

In the exemplary stent described above, a number of examples may be utilized to illustrate the relationship of equiaxed granularity to wall thickness. In the first example, the wall thickness may be varied in the range from about 0.013 mm (0.0005 inches) to about 0.152 mm (0.006 inches) for a stent having an expanded inside diameter of less than about 2.5 millimeters. Accordingly, for a maximal number of equiaxed grains, which in the exemplary embodiment is substantially not more than ten (10) discrete grains across the thickness of the wall, the equiaxed grain-size shall be equal to or greater than substantially 1.25 $\mu$m (1.25 microns). This dimensional attribute may be arrived at by simply dividing the minimal available wall thickness by the maximal number of available equiaxed grains. In another example, the wall thickness may be varied in the range from about 0.051 mm (0.002 inches) to about 0.203 mm (0.008 inches) for a stent having an expanded inside diameter from about 2.5 millimeters to about 5.0 millimeters. Accordingly, for a maximal number of equiaxed grains, which in the exemplary embodiment is substantially not more than ten (10) discrete grains across the thickness of the wall, the equiaxed grain-size shall be equal to or greater than substantially 5.0 $\mu$m (5.0 microns). In yet another example, the wall thickness may be varied in the range from about 0.102 mm (0.004 inches) to about 0.030 mm (0.012 inches) for a stent having an expanded inside diameter from about 5.0 millimeters to about 12.0 millimeters. Accordingly, for a maximal number of equiaxed grains, which in the exemplary embodiment is substantially not more than ten (10) discrete grains across the thickness of the wall, the equiaxed grain-size shall be equal to or greater than substantially 10 $\mu$m (10.0 microns). In yet still another example, the wall thickness may be varied in the range from about 0.152 mm (0.006 inches) to about 0.635 mm (0.025 inches) for a stent having an expanded inside diameter from about 12.0 millimeters to about 50.0 millimeters. Accordingly, for a maximal number of equiaxed grains, which in the exemplary embodiment is substantially not more than ten (10) discrete grains across the thickness of the wall, the equiaxed grain-size shall be equal to or greater than substantially 15.0 $\mu$m (15.0 microns). In making the above calculations, it is important to maintain rigorous consistency of dimensional units.

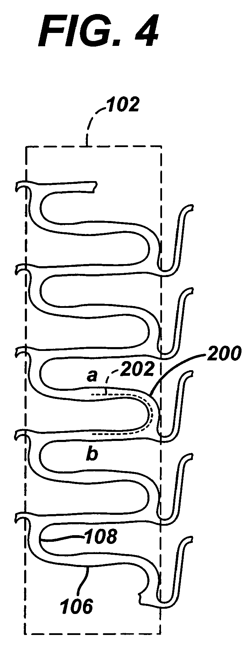

In accordance with another aspect of the present invention, the elements of the exemplary stent 100, illustrated in Figure 3, may be further defined in terms that may be utilized to describe the relationship between geometry, material and the effects of applied loading. Referring to Figure 4, there is illustrated, in planar view, a single hoop component 102. As described above, the hoop component 102 is formed as a series of substantially circumferentially oriented radial strut members 106 and alternating radial arc members 108. However, the hoop component 102 may also be defined as a number of interconnected loops, wherein a single loop is the element between point a and point b in Figure 4. In other words, each single loop comprises a portion of two radial strut members and an entire radial arc member. Formulaically, the linear length of a single loop, $L_L$, may be given by

$$L_L = RS_L + RA_L, \tag{2}$$

wherein $RS_L$ is the length of a strut member and $RA_L$ is the linear length of the arc member as measured through its center line. Given that the hoop 102 may be defined as a number of interconnected loops, the total linear path length of a hoop, $H_L$, may be given by

$$H_L = \Sigma L_L. \tag{3}$$

From the expressions represented by equations (2) and (3) a number of ratios may be developed that describe or define the relationship between geometry, material and the effects of applied load. More specifically, it is the unique material composition and built in properties, i.e. microstructure, that provide the means for fabricating a stent with various geometries that are able to withstand the various loading conditions as is described in detail subsequently. For example, a stent may be designed such that each radial strut's member is configured to exhibit substantially no permanent plastic deformation upon expansion while each radial arc member is configured to accommodate substantially all permanent plastic deformation upon expansion. Alternately, a stent may be designed such that each radial arc member is configured to exhibit substantially no permanent plastic deformation upon expansion, while each radial strut member is configured to accommodate substantially all permanent deformation upon expansion. As these two examples represent the two extremes, it is important to note that the present invention also applies to the continuum between these extremes.

The material properties that are of importance relate to the microstructure as described in detail above. Specifically, the stents are fabricated from a metallic material processed to have a microstructure with a granularity of about thirty-two microns or less and comprise from about two to about ten substantially equiaxed grains as measured across the wall thickness of the stent. The ratios set forth below help describe the desirable properties of the stent.

The expansion efficiency ratio, $H_{eff}$, is given by

$$H_{eff} = C/H_L, \tag{4}$$

wherein C is the circumference of a fully expanded hoop (or stent) and $H_L$ is the total path length of a hoop as set forth in equation (3). Due to the metallic materials and associated built-in properties thereof, the ratio of equation (4) that may be achieved is given by

$$H_{eff} = C/H_L > 0.25. \tag{5}$$

In other words, the ratio of the circumference of a fully expanded hoop to the total path of the hoop is greater than 0.25. Obviously, the maximum that this ratio may achieve is unity since the path length should not be greater than the circumference of the expanded hoop. However, it is this 0.25 expansion efficiency ratio that is important. In any stent design it is desirable to minimize the amount of structural metal within the vessel and to reduce the overall complexity of fabrication. Expansion efficiency ratios of greater than 0.25 are achievable through the utilization of these new materials. It is important to note that the circumference of a fully expanded hoop should substantially correspond to the normal luminal circumference of the vessel into which the stent is placed. In addition, if the lumen of the vessel is not substantially circular, perimeter may be substituted for circumference, C.

The loop efficiency ratio, $L_{eff}$, is given by

$$L_{eff} = L_L / RA_L, \tag{6}$$

wherein $L_L$ is the linear length or path-length of a single loop given by equation (2) and $RA_L$ is the linear length or path-length of an arc member. Using the elementary rules of algebraic substitution while maintaining rigorous dimensional integrity, Equation (6) may be rewritten as

$$L_{eff} = (RS_L + RA_L) / RA_L. \tag{7}$$

As may be easily seen from Equation (7), the loop efficiency ratio may never be less than unity. However, because of the material properties, the linear length or path-length of the arc and the linear length or path-length of the struts may be manipulated to achieve the desired characteristics of the final product. For example, under the condition where the strain is primarily carried within the radial arc member, increasing the length of the radial strut for a fixed expansion diameter (displacement controlled phenomena) reduces the magnitude of the non-recoverable plastic strain integrated across the entirety of the radial arc. Similarly, under the condition where the strain is primarily carried within the radial strut member, increasing the length of the radial strut for a fixed expansion diameter (displacement controlled phenomena) reduces the magnitude of the non-recoverable plastic strain integrated across the entirety of the radial strut. In addition, under the condition where the strain is primarily carried within the radial arc member, increasing the path-length of the radial arc for a fixed expansion diameter (displacement controlled phenomena) reduces the magnitude of the non-recoverable plastic strain integrated across the entirety of the radial arc. As these examples represent the extremes, it is important to note that the present invention also applies to the continuum between these extremes.

Accordingly, since the material is able to withstand greater loading, various designs based upon the above ratios may be achieved.

It is important to note that no assumption is made as to the symmetry of the radial struts or radial arc that comprise each single loop and the hoops of the structure. Furthermore, these principals also apply to loops that are interconnected along the longitudinal axis but not necessarily along the radial axis, for example, loops configured into a helical structure. Although a single loop has been illustrated with a single arc member, it obvious to those of ordinary skill in the art, a single loop may be comprise no radial arcs, a single radial arc (as illustrated in Figures 3 and 4) and/or multiple radial arcs and no radial strut, a single radial strut and/or multiple radial struts (as illustrated in Figure 3 and 4).

Intraluminal scaffolds or stents may comprise any number of design configurations and materials depending upon the particular application and the desired characteristics. One common element of all stent designs is that each stent comprises at least one load-bearing element. Typically, the load-bearing elements have well defined geometries; however, alternate non-conventional geometries may be described in-terms of a bounded cross-sectional area. These bounded areas may be engineered to have either an asymmetric or symmetric configuration. Regardless of the configuration, any bounded cross-sectional area should include at least one internal grain boundary. Those skilled in the art will recognize that the grain-boundary identified in this exemplary embodiment should preferably not constitute any measurable degree of the surface defined by the perimeter of the bounded cross-sectional area. Additionally, those skilled in the art will understand that the grain-boundary discussed in this exemplary embodiment should preferably be characterized as having a high-angle (i.e. typically greater than or equal to about 35 degrees) crystallographic interface. Also, in the presence of microstructural defects such as microcracks (i.e. lattice level discontinuities that can be char-acterized as planar crystallographic defects), the fatigue crack growth-rate will be expected to be proportional to the number of grains that exist within the bounded cross-sectional area. Since there is one internal grain boundary, this ensures that at least two discrete grains or portions thereof will exist within the bounded cross-sectional area. As described herein, the well-known Hall-Petch relationship that inversely relates grain-size to strength should be observed in this exemplary embodiment as the average grain-size will proportionally decrease as the number of grains within the bounded cross-sectional area increases. In addition, as the number of grains increase within the bounded cross-sectional area, the ability for the microstructure to internally accommodate stress-driven grain boundary sliding events will also increase and should preferably increase localized ductility.

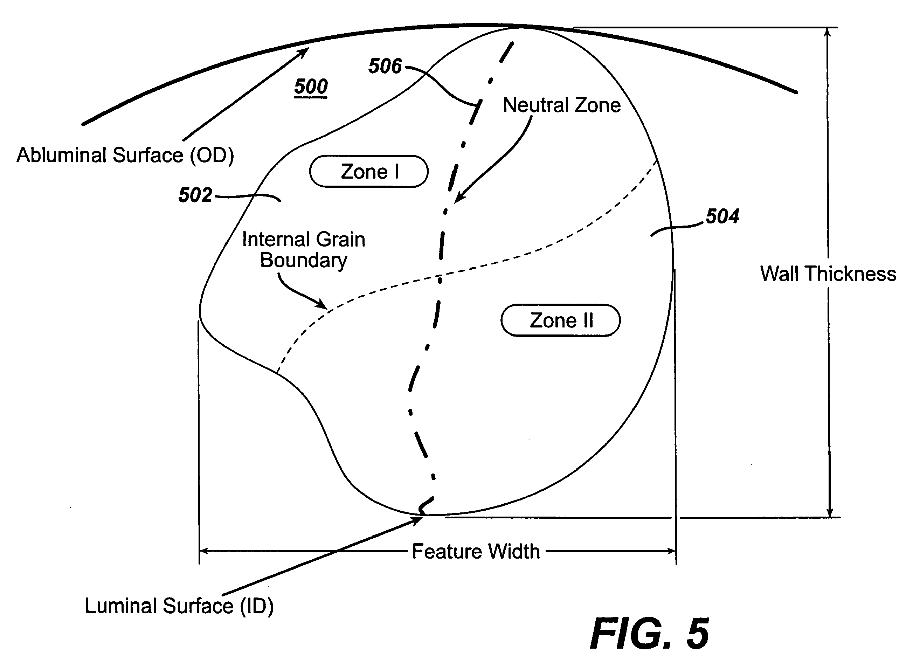

Referring to Figure 5, there is illustrated a cross-sectional representation of a load-bearing stent element 500. As shown, the bounded cross-sectional area comprises a first zone 502, a second zone 504 and a neutral zone 506 which are the result of a stress gradient that is directly proportional to the external loading conditions. The neutral zone 506 is generally defined as a substantially stress free zone that exists between and is bounded by the first zone 502 and the second zone 504. As a function of changing external loading conditions either from the unloaded condition or a loaded condition, the first and second zones, 502 and 504, will undergo a change in tensile and/or compressive stress. It is important to note that the zone assignments shown in Figure 5 are illustrative in nature and not intended to define relative positioning within the bounded area. The load bearing stent element 500 has a wall thickness that is defined as the radial distance between the luminal surface and the abluminal surface. The load bearing element 500 also has a feature width. The feature width is defined as the linear distance across the first zone 502, neutral zone 506 and the second zone 504 in the direction that is substantially orthogonal to the wall thickness. It is important to note that the feature width is measured at a point that represents the greatest measurable distance in a direction that is substantially orthogonal to the wall thickness.

Other elements of the intraluminal scaffold may be designed in a similar manner, for example, the flexible connectors. While not considered the primary load bearing elements, the flexible connectors undergo longitudinally applied external loading and applied external bending moments.

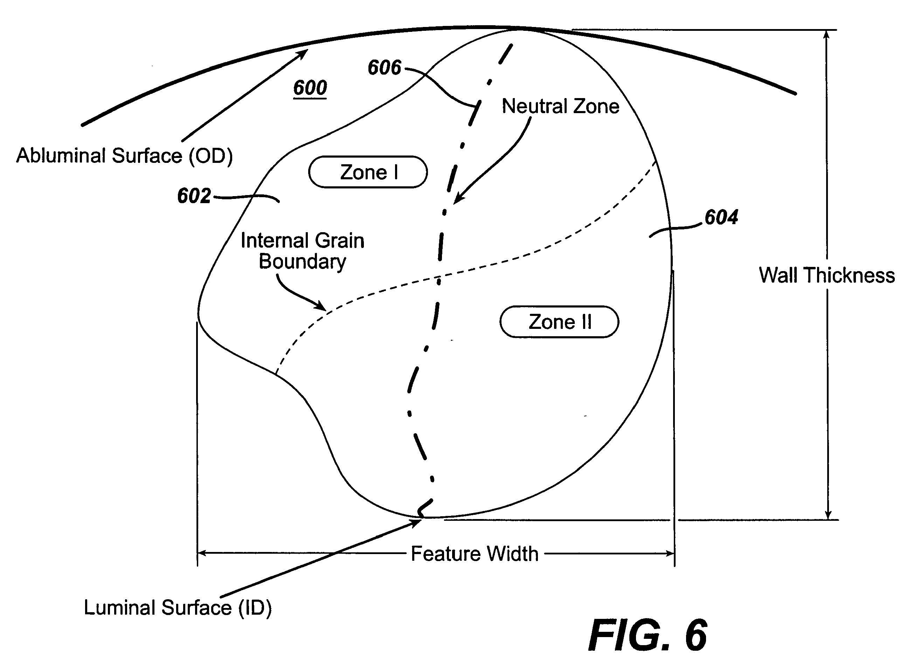

Referring to Figure 6, there is illustrated a cross-sectional representation of a flexible connector stent element 600. The flexible connector stent element interconnects the substantially radial load-bearing stent elements or hoop components. The flexible connector stent elements are substantially oriented along the longitudinal axis of the stent. Referring back to Figure 3, the flexible connector stent elements comprise the flexible connectors 104 which are formed from a continuous series of substantially longitudinally oriented flexible strut members 112 and alternating flexible arc members 114. It is important to note the flexible connector stent elements may comprise a simpler design than described herein, for example, a singular longitudinal oriented strut or arc. As shown, under substantially longitudinal applied external loading conditions, i.e., tensile and compressive the bounded cross-sectional area comprises a first zone 602, a second zone 604 and a neutral zone 606 which are the result of a stress gradient that is directly proportional to these external loading conditions. The neutral zone 606 is generally defined as a substantially stress free zone that exists between and is bounded by the first zone 602 and the second zone 604. As a function of changing external loading conditions either from the unloaded condition or a loaded condition, the first and second zones, 602 and 604, will undergo a change in tensile and/or compressive stress. It is important to note that the zone assignments shown in Figure 6 are illustrative in nature and not intended to define relative positioning within the bounded area. The flexible connector stent element 600 has a wall thickness that is defined as the radial distance between the luminal surface and the abluminal surface. The flexible connector element 600 also has a feature width. The feature width is defined as the linear distance that is substantially orthogonal to the wall thickness. It is important to note that the feature width is measured at a point that represents the greatest measurable distance in a direction that is substantially orthogonal to the wall thickness.

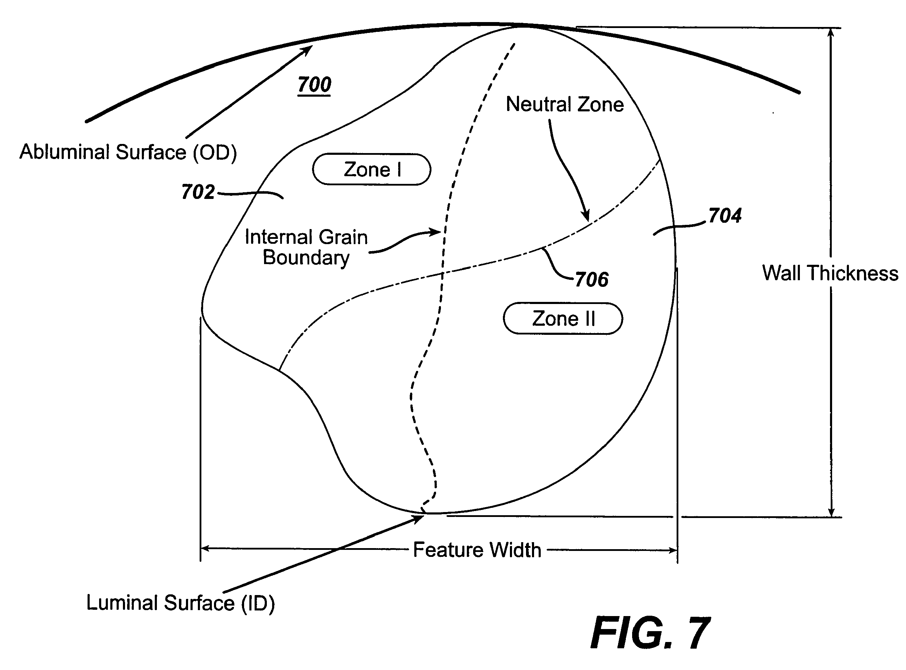

Referring to Figure 7, there is illustrated another cross-sectional representation of a flexible connector stent element 700. As shown, under external loading conditions that are substantially comprised of applied bending moments, the bounded cross-sectional area comprises a first zone 702, a second zone 704 and a neutral zone 706 which are the result of a stress gradient that is directly proportional to these external loading conditions. The neutral zone 706 is generally defined as a substantially stress free zone that exists between and is bounded by the first zone 702 and the second zone 704. As a function of changing external loading conditions either from the unloaded condition or a loaded condition, the first and second zones, 702 and 704, will undergo a change in tensile and/or compressive stress. It is important to note that the zone assignments shown in Figure 7 are illustrative in nature and not intended to define relative positioning within the bounded area. The flexible connector stent element 700 has a wall thickness that is defined as the radial distance between the luminal surface and the abluminal surface. The flexible connector element 700 also has a feature width. The feature width is defined as the linear distance that is substantially orthogonal to the wall thickness. It is important to note that the feature width is measured at a point that represents the greatest measurable distance in a direction that is substantially orthogonal to the wall thickness.

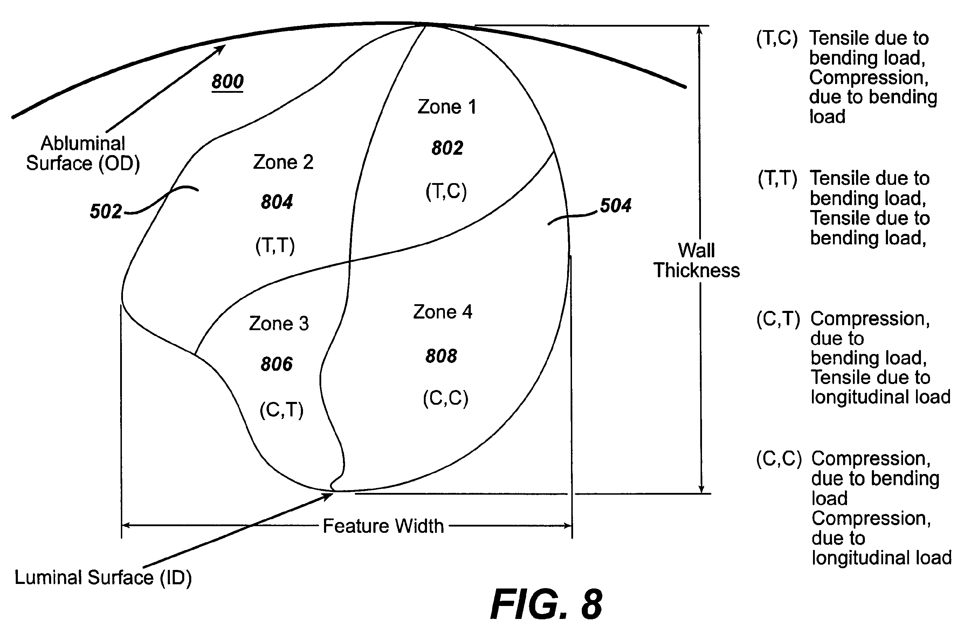

Referring to Figure 8, there is yet another illustrated cross-sectional representation of a flexible connector stent element 800. As shown, under external loading conditions that are comprised of blend of applied bending moments and longitudinal applied external loading conditions, the bounded cross-sectional area comprises a first zone 802, a second zone 804, a third zone 806, a fourth zone 808 and an equilibrium zone (not illustrated) which are the result of one or more stress gradients that are directly proportional to these external loading conditions. The equilibrium zone is generally defined as a substantially stress free zone that exists between and is bounded by at least two zones. As a function of changing external loading conditions either from the unloaded condition or a loaded condition, the zones, 802, 804, 806 and/or 808 will undergo changes in tensile and/or compressive stress. It is important to note that the zone assignments shown in Figure 8 are illustrative in nature and not intended to define relative positioning within the bounded area. The flexible connector stent element 800 has a wall thickness that is defined as the radial distance between the luminal surface and the abluminal surface. The flexible connector element 800 also has a feature width. The feature width is defined as the linear distance that is substantially orthogonal to the wall thickness. It is important to note that the feature width is measured at a point that represents the greatest measurable distance in a direction that is substantially orthogonal to the wall thickness.

The exemplary load bearing stent element 500 and the flexible connector stent elements 600, 700 and 800 that are illustrated in Figures 5, 6, 7 and 8 may be fabricated from any of the metallic materials described herein and processed to preferably exhibit a multiplicity of grains when measured across the bounded cross-sectional area defined by the wall thickness and the feature width. When fabricated from a substantially polymeric material system, the properties and attributes described above, that are recognizable by one of appropriate skill and technical qualification in the relevant art, may be utilized to produce a load-bearing structure that is substantially similar to that created with the metallic materials described above.

Accordingly, in yet another exemplary embodiment, an intraluminal scaffold element may be fabricated from a non-metallic material such as a polymeric material including non-crosslinked thermoplastics, cross-linked thermosets, composites and blends thereof. There are typically three different forms in which a polymer may display the mechanical properties associated with solids; namely, as a crystalline structure, as a semicrystalline structure and/or as an amorphous structure. All polymers are not able to fully crystallize, as a high degree of molecular regularity within the polymer chains is essential for crystallization to occur. Even in polymers that do substantially crystallize, the degree of crystallinity is generally less than 100 percent. Within the continuum between fully crystalline and amorphous structures, there are two thermal transitions possible; namely, the crystal-liquid transition (i.e. melting point temperature, $T_m$) and the glass-liquid transition (i.e. glass transition temperature, $T_g$). In the temperature range between these two transitions there may be a mixture of orderly arranged crystals and chaotic amorphous polymer domains.

The Hoffman-Lauritzen theory of the formation of polymer crystals with "folded" chains owes its origin to the discovery in 1957 that thin single crystals of polyethylene may be grown from dilute solutions. Folded chains are preferably required to form a substantially crystalline structure. Hoffman and Lauritzen established the foundation of the kinetic theory of polymer crystallization from "solution" and "melt" with particular attention to the thermodynamics associated with the formation of chain-folded nuclei.

Crystallization from dilute solutions is required to produce single crystals with macroscopic perfection (typically magnifications in the range of about 200x to about 400x). Polymers are not substantially different from low molecular weight compounds such as inorganic salts in this regard. Crystallization conditions such as temperature, solvent and solute concentration may influence crystal formation and final form. Polymers crystallize in the form of thin plates or "lamellae." The thickness of these lamellae is on the order of 10 nanometers (i.e. nm). The dimensions of the crystal plates perpendicular to the small dimensions depend on the conditions of the crystallization but are many times larger than the thickness of the platelets for a well-developed crystal. The chain direction within the crystal is along the short dimension of the crystal, which indicates that, the molecule folds back and forth (e.g. like a folded fire hose) with successive layers of folded molecules resulting in the lateral growth of the platelets. A crystal does not consist of a single molecule nor does a molecule reside exclusively in a single crystal. The loop formed by the chain as it emerges from the crystal turns around and reenters the crystal. The portion linking the two crystalline sections may be considered amorphous polymer. In addition, polymer chain ends disrupt the orderly fold patterns of the crystal, as described above, and tend to be excluded from the crystal. Accordingly, the polymer chain ends become the amorphous portion of the polymer. Therefore, no currently known polymeric material can be 100 percent crystalline. Post polymerization processing conditions dictate the crystal structure to a substantial extent.

Single crystals are not observed in crystallization from bulk processing. Bulk crystallized polymers from melt exhibits domains called "spherulites" that are symmetrical around a center of nucleation. The symmetry is perfectly circular if the development of the spherulite is not impinged by contact with another expanding spherulite. Chain folding is an essential feature of the crystallization of polymers from the molten state. Spherulites are composed of aggregates of "lamellar" crystals radiating from a nucleating site. Accordingly, there is a relationship between solution and bulk grown crystals.

The spherical symmetry develops with time. Fibrous or lathlike crystals begin branching and fanning out as in dendritic growth. As the lamellae spread out dimensionally from the nucleus, branching of the crystallites continue to generate the spherical morphology. Growth is accomplished by the addition of successive layers of chains to the ends of the radiating laths. The chain structure of polymer molecules suggests that a given molecule may become involved in more than one lamella and thus link radiating crystallites from the same or adjacent spherulites. These interlamellar links are not possible in spherulites of low molecular weight compounds, which show poorer mechanical strength as a consequence.

The molecular chain folding is the origin of the "Maltese" cross, which identifies the spherulite under crossed polarizers. For a given polymer system, the crystal size distribution is influenced by the initial nucleation density, the nucleation rate, the rate of crystal growth, and the state of orientation. When the polymer is subjected to conditions in which nucleation predominates over radial growth, smaller crystals result. Larger crystals will form when there are relatively fewer nucleation sites and faster growth rates. The diameters of the spherulites may range from about a few microns to about a few hundred microns depending on the polymer system and the crystallization conditions.

Therefore, spherulite morphology in a bulk-crystallized polymer involves ordering at different levels of organization; namely, individual molecules folded into crystallites that in turn are oriented into spherical aggregates. Spherulites have been observed in organic and inorganic systems of synthetic, biological, and geological origin including moon rocks and are therefore not unique to polymers.

Stress induced crystallinity is important in film and fiber technology. When dilute solutions of polymers are stirred rapidly, unusual structures develop which are described as having "shish kebab" morphology. These consist of chunks of folded chain crystals strung out along a fibrous central column. In both the "shish" and the "kebab" portions of the structure, the polymer chains are parallel to the overall axis of the structure.

When a polymer melt is sheared and quenched to a thermally stable condition, the polymer chains are perturbed from their random coils to easily elongate parallel to the shear direction. This may lead to the formation of small crystal aggregates from deformed spherulites. Other morphological changes may occur, including spherulite to fibril transformation, polymorphic crystal formation change, reorientation of already formed crystalline lamellae, formation of oriented crystallites, orientation of amorphous polymer chains and/or combinations thereof.

It is important to note that polymeric materials may be broadly classified as synthetic, natural and/or blends thereof. Within these broad classes, the materials may be defined as biostable or biodegradable. Examples of biostable polymers include polyolefins, polyamides, polyesters, fluoropolymers, and acrylics. Examples of natural polymers include polysaccharides and proteins. Examples of biodegradable polymers include the family of polyesters such as polylactic acid, polyglycolic acid, polycaprolactone, polytrimethylene carbonate and polydioxanone. Additional examples of biodegradable polymers include polyhydroxalkanoates such as polyhydroxybutyrate-co-valerates; polyanhydrides; polyorthoesters; polyaminoacids; polyesteramides; polyphosphoesters; and polyphosphazenes. Copolymers and blends of any of the described polymeric materials may be utilized in accordance with the present invention.

When constructing an intraluminal stent from metallic materials, a maximum granularity of about 32 microns or less was necessary to achieve the functional properties and attributes described herein. When constructing an intraluminal stent from polymeric materials, a maximum spherulitic size of about 50 microns or less was necessary to achieve the functional properties and attributes described herein.

**Claims**

1.  An intraluminal scaffold comprising at least one flexible connector element having a luminal surface and an abluminal surface, the flexible connector element having a predetermined wall thickness, wherein the wall thickness is defined by the radial distance between the luminal surface and the abluminal surface, and a predetermined feature width, wherein an area bounded by the wall thickness and the feature width comprises a plurality of zones undergoing at least one of tensile, compressive or substantially zero stress change due to external loading, the flexible connector element being fabricated from a metallic material processed to have a microstructure with a granularity of about 32 microns or less and at least one internal grain boundary within the bounded area.

2.  The intraluminal scaffold according to claim 1, wherein the metallic material is formed from a solid-solution alloy comprising chromium in the range from about 10 weight percent to about 30 weight percent, tungsten in the range from about 5 weight percent to about 20 weight percent, nickel in the range from about 5 weight percent to about 20 weight percent, manganese in the range from about 0 weight percent to about 5 weight percent, carbon in the range from about 0 weight percent to about 1 weight percent, iron in an amount not to exceed 0.12 weight percent, silicon in an amount not to exceed 0.12 weight percent, phosphorus in an amount not to exceed 0.04 weight percent, sulfur in an amount not to exceed 0.03 weight percent and the remainder cobalt.

3.  The intraluminal scaffold according to claim 1, wherein the metallic material is formed from a solid-solution alloy comprising chromium in the range from about 10 weight percent to about 30 weight percent, tungsten in the range from about 5 weight percent to about 20 weight percent, nickel in the range from about 5 weight percent to about 20 weight percent, manganese in the range from about 0 weight percent to about 5 weight percent, carbon in the range from about 0 weight percent to about 1 weight percent, iron in an amount not to exceed 0.12 weight percent, silicon in an amount not to exceed 0.4 weight percent, phosphorus in an amount not to exceed 0.04 weight percent, sulfur in an amount not to exceed 0.03 weight percent and the remainder cobalt.

4.  The intraluminal scaffold according to claim 1, wherein the metallic material is formed from a solid-solution alloy comprising chromium in the range from about 10 weight percent to about 30 weight percent, tungsten in the range from about 5 weight percent to about 20 weight percent, nickel in the range from about 5 weight percent to about 20 weight percent, manganese in the range from about 0 weight percent to about 5 weight percent, carbon in the range from about 0 weight percent to about 1 weight percent, iron in an amount not to exceed 3 weight percent, silicon in an amount not to exceed 0.12 weight percent, phosphorus in an amount not to exceed 0.04 weight percent, sulfur in an amount not to exceed 0.03 weight percent and the remainder cobalt.

5.  The intraluminal scaffold according to claim 1, wherein the metallic material is formed from a solid-solution alloy comprising nickel in the range from about 20 weight percent to about 24 weight percent, chromium in the range from about 21 weight percent to about 23 weight percent, tungsten in the range from about 13 weight percent to about 15 weight percent, manganese in the range from about 0 weight percent to about 1.25 weight percent, carbon in the range from about 0.05 weight percent to about 0.15 weight percent, lanthanum in the range from about 0.02 weight percent to about 0.12 weight percent, boron in the range from about 0 weight percent to about 0.015 weight percent, iron in an amount not to exceed 0.12 weight percent, silicon in an amount not to exceed 0.12 weight percent and the remainder cobalt.

6.  The intraluminal scaffold according to claim 1, wherein the metallic material is formed from a solid-solution alloy comprising nickel in the range from about 20 weight percent to about 24 weight percent, chromium in the range from about 21 weight percent to about 23 weight percent, tungsten in the range from about 13 weight percent to about 15 weight percent, manganese in the range from about 0 weight percent to about 1.25 weight percent, carbon in the range from about 0.05 weight percent to about 0.15 weight percent, lanthanum in the range from about 0.02 weight percent to about 0.12 weight percent, boron in the range from about 0 weight percent to about 0.015 weight percent, silicon in the range from about 0.2 weight percent to about 0.5 weight percent, iron in an amount not to exceed 0.12 weight percent and the remainder cobalt.

7.  The intraluminal scaffold according to claim 1, wherein the metallic material is formed from a solid-solution alloy comprising nickel in the range from about 20 weight percent to about 24 weight percent, chromium in the range from about 21 weight percent to about 23 weight percent, tungsten in the range from about 13 weight percent to about 15 weight percent, iron in the range from about 0 weight percent to about 3 weight percent, manganese in the range from about 0 weight percent to about 1.25 weight percent, carbon in the range from about 0.05 weight percent to about 0.15 weight percent, lanthanum in the range from about 0.02 weight percent to about 0.12 weight percent, boron in the range from about 0 weight percent to about 0.015 weight percent, silicon in an amount not to exceed 0.12 weight percent and the remainder cobalt.

8. The intraluminal scaffold according to claim 1, wherein the metallic material is formed from a solid-solution alloy comprising nickel in the range from about 33 weight percent to about 37 weight percent, chromium in the range from about 19 weight percent to about 21 weight percent, molybdenum in the range from about 9 weight percent to about 11 weight percent, iron in the range from about 0 weight percent to about 1 weight percent, manganese in the range from about 0 weight percent to about 0.15 weight percent, silicon in the range from about 0 weight percent to about 0.15 weight percent, carbon in the range from about 0 to about 0.025 weight percent, phosphorous in the range from about 0 to about 0.015 weight percent, boron in the range from about 0 to about 0.015 weight percent, sulfur in the range from about 0 to about 0.010 weight percent, titanium in an amount not to exceed 0.015 weight percent and the remainder cobalt.

9. The intraluminal scaffold according to claim 1, wherein the metallic material formed from a biocompatible, solid-solution alloy comprising chromium in the range from about 26 weight percent to about 30 weight percent, molybdenum in the range from about 5 weight percent to about 7 weight percent, nickel in the range from about 0 weight percent to about 1 weight percent, silicon in the range from about 0 weight percent to about 1 weight percent, manganese in the range from about 0 weight percent to about 1 weight percent, iron in the range from about 0 weight percent to about 0.75 weight percent, nitrogen in the range from about 0 to about 0.25 weight percent, carbon in an amount not to exceed 0.025 weight percent and the remainder cobalt.

10. An intraluminal scaffold comprising at least one flexible connector element having a luminal surface and an abluminal surface, the flexible connector element having a predetermined wall thickness, wherein the wall thickness is defined by the radial distance between the luminal surface and the abluminal surface, and a predetermined feature width, wherein an area bounded by the wall thickness and the feature width comprises a plurality of zones undergoing at least one of tensile, compressive or substantially zero stress change due to external loading, the flexible connector element being fabricated from a material processed to have a microstructure with structural domains having a size of about 50 microns or less and at least one internal structural boundary within the bounded area.

11. The intraluminal scaffold according to claim 10, wherein the material is formed from a synthetic polymeric material.

12. The intraluminal scaffold according to claim 11, wherein the synthetic polymeric material comprises polyolefins.

13. The intraluminal scaffold according to claim 11, wherein the synthetic polymeric material comprises polyamides.

14. The intraluminal scaffold according to claim 11, wherein the synthetic polymeric material comprises polyesters.

15. The intraluminal scaffold according to claim 11, wherein the synthetic polymeric material comprises fluoropoloymers.

16. The intraluminal scaffold according to claim 10, wherein the material is formed a natural polymeric material.

17. The intraluminal scaffold according to claim 16, wherein the natural polymeric material comprises polysacaccharides.

18. The intraluminal scaffold according to claim 16, wherein the natural polymeric material comprises proteins.

19. The intraluminal scaffold according to claim 10, wherein the material is formed from a synthetic biodegradable polymeric material.

20. The intraluminal scaffold according to claim 19, wherein the biodegradable polymeric material comprises polyesters.

21. The intraluminal scaffold according to claim 19, wherein the biodegradable polymeric material comprises polyhydroxalkanoates.

22. The intraluminal scaffold according to claim 19, wherein the biodegradable polymeric material comprises polyanhydrides.

23. The intraluminal scaffold according to claim 19, wherein the biodegradable polymeric material comprises polyorthoesters.

**24.** The intraluminal scaffold according to claim 19, wherein the biodegradable polymeric material comprises polyaminoacids.

**25.** The intraluminal scaffold according to claim 19, wherein the biodegradable polymeric material comprises polyesteramides.

**26.** The intraluminal scaffold according to claim 19, wherein the biodegradable polymeric material comprises polyphosphoesters.

**27.** The intraluminal scaffold according to claim 19, wherein the biodegradable polymeric material comprises polyphosphazenes.

**28.** The intraluminal scaffold according to claim 10, wherein the domains comprise spherulitic structures.

**29.** The intraluminal scaffold according to claim 10, wherein the domains comprise folded-chain structures.

**30.** The intraluminal scaffold according to claim 10, wherein the domains comprise spherulitic and folded-chain structures.

# FIG. 1

# FIG. 2

EP 1 674 118 A2

# FIG. 3

# FIG. 4

Wall Thickness

Abluminal Surface (OD)

Neutral Zone

Zone I

Zone II

Internal Grain Boundary

Feature Width

Luminal Surface (ID)

500

502

504

506

**FIG. 5**

FIG. 6

EP 1 674 118 A2

**FIG. 7**

Abluminal Surface (OD)

700

702

Internal Grain Boundary

Zone I

Neutral Zone

Zone II

704

706

Wall Thickness

Feature Width

Luminal Surface (ID)

EP 1 674 118 A2

FIG. 8

(T,C) Tensile due to bending load, Compression, due to bending load

(T,T) Tensile due to bending load, Tensile due to bending load,

(C,T) Compression, due to bending load, Tensile due to longitudinal load

(C,C) Compression, due to bending load Compression, due to longitudinal load